# EUROPÄISCHE PATENTSCHRIFT

(11) **EP 0 483 632 B1**
(45) Veröffentlichungstag und Bekanntmachung des Hinweises auf die Patenterteilung: **24.05.1995**
(21) Anmeldenummer: 91117897.8
(22) Anmeldetag: 21.10.1991
(51) Int. Cl.: C07C 68/02, C07C 69/96

(54) **Verfahren zur Herstellung von Arylcarbonaten**
Process for the preparation of aryl carbonates
Procédé de préparation de carbonates d'aryle

(30) Priorität: 01.11.1990 DE 4034717
(43) Veröffentlichungstag der Anmeldung: 06.05.1992
(73) Patentinhaber: BAYER AG, 51368 Leverkusen (DE)
(72) Erfinder: Buysch, Hans-Josef, Dr., W-4150 Krefeld (DE); Schön, Norbert, Dr., W-4150 Krefeld (DE); Jeromin, Günther, Dr., W-4150 Krefeld (DE)

(56) Entgegenhaltungen:
- DE-A- 2 161 254

## Beschreibung

Die vorliegende Erfindung betrifft ein Verfahren zur Herstellung von Carbonaten mit aromatischen Estergruppen durch Umsetzung von aromatischen Monohydroxyverbindungen mit Phosgen oder Chlorkohlensäureestern aromatischer Monohydroxyverbindungen unter Abspaltung von Chlorwasserstoff in Gegenwart von Aktivkohle.

Carbonate mit aromatischen Estergruppen eignen sich zur Herstellung von Polycarbonaten nach dem Schmelzumesterungsverfahren, zur Herstellung von Phenylurethanen oder sind Vorprodukte zur Herstellung von Wirkstoffen auf dem Pharma- oder Pflanzenschutzsektor.

Es ist bekannt, daß die Arylcarbonate durch Phasengrenzflächenphosgenierung (Schotten-Baumann-Reaktion) von aromatischen Hydroxyverbindungen gewonnen werden können. Dabei wirkt sich die Verwendung von Lösungsmitteln und Natronlauge nachteilig aus, da durch die Lauge eine teilweise Verseifung von Phosgen oder Chlorkohlensäureester stattfinden kann. In jedem Falle bringen die großen Mengen von anfallendem Natriumchlorid eine Abwasserbelastung mit sich. Ferner muß Sorge für die Lösungsmittel-Rückgewinnung getragen werden, wobei ein effektiver Umweltschutz gesichert sein muß.

Man hat daher eine Kondensation ohne Mitverwendung von Lösungsmitteln in Gegenwart von Tetramethylammoniumhalogeniden als Katalysatoren vorgeschlagen (US-PS 2.837.555). Dabei sind aber die benötigten Katalysatormengen relativ groß. Man muß in der Regel mit 5 - 7 Gewichtsprozent Katalysator, bezogen auf die eingesetzte Phenolmenge, arbeiten, um wirtschaftliche Reaktionsgeschwindigkeiten zu erhalten; die Reaktionstemperaturen von 180°C - 215°C bringen die Gefahr einer Zersetzung der thermolabilen Tetramethylammoniumhalogenide mit sich. Der Katalysator muß ferner anschließend durch Waschen mit Wasser entfernt werden, wodurch seine Rückgewinnung erheblich erschwert wird. Darüber hinaus wird weit über die stöchiometrisch notwendige Menge an Phosgen verbraucht. Die Ausbeuten an Diphenylcarbonat betragen nur wenig mehr als 80 % der theroretischen Ausbeute.

Nach einem weiteren Verfahren (US-PS 3.234.263) werden Diphenylcarbonate durch Erhitzen von Phenylchlorkohlensäureestern in Gegenwart großer Mengen (Erd)Alkaliverbindungen und tertiären Stickstoffbasen als Katalysatoren erhalten. Dieses Verfahren hat jedoch den Nachteil, daß man hohe Temperaturen anwenden muß, um auch nur annähernd auf wirtschaftlich vertretbare Reaktionszeiten zu kommen. Bei diesem Verfahren geht die Hälfte des ursprünglich eingesetzten Phosgens in Form von CO₂ verloren. Außerdem muß man in einem vorgelagerten separaten Verfahrensschritt die Chlorkohlensäureester synthetisieren.

In DE-OS 24 47 348 wird ein Verfahren zur Herstellung von Arylcarbonaten vorgeschlagen, wonach eine Phosgenierung von Phenolen in Gegenwart von heterocyclischen Stickstoffbasen durchgeführt wird. Obwohl gegenüber den vorgenannten Verfahren bessere Ausbeuten bei einfacherer Verfahrensweise erhalten werden, bleiben die Schwierigkeiten einer sauberen Abtrennung des Katalysators und es bleibt weiterhin der Wunsch nach einer einfachen kontinuierlichen Arbeitsweise.

Es wurde nun gefunden, daß man Carbonate mit Arylgruppen in höchst einfacher Weise gewinnen kann, wenn man Phenole bei erhöhter Temperatur in Gegenwart von Aktivkohle als Katalysator mit Phosgen oder Chlorkohlensäureestern umsetzt.

Der Gegenstand der vorliegenden Erfindung ist demnach ein Verfahren zur Herstellung von Arylcarbonaten durch Umsetzung von aromatischen Monohydroxyverbindungen mit Phosgen oder Chlorkohlensäureestern aromatischer Monohydroxyverbindungen, das dadurch gekennzeichnet ist, daß man bei einer Temperatur im Bereich von 50 - 350°C in Gegenwart von Aktivkohle als Katalysator arbeitet.

Das erfindungsgemäße Verfahren hat den großen Vorteil, daß man den Katalysator sehr leicht abtrennen kann und keine Verunreinigungen im rohen Reaktionsprodukt verbleiben. Die Aufarbeitung wird dadurch wesentlich vereinfacht.

Aromatische Monohydroxyverbindungen für das erfindungsgemäße Verfahren sind solche der Formel

Ar¹-OH (I),

worin
- Ar¹: Phenyl, Naphtyl, Anthryl, Phenanthryl, Indanyl, Tetrahydronaphthyl oder den Rest eines 5- oder 6-gliedrigen aromatischen Heterocyclus mit 1 oder 2 Heteroatomen aus der Gruppe von N, O und S bedeutet, wobei diese isocyclischen oder heterocyclischen Reste durch 1 oder 2 Substituenten aus der Gruppe von geradkettigem oder verzweigtem C₁-C₄-Alkyl, geradkettigem oder verzweigtem C₁-C₄-Alkoxy, Phenyl, Cyano und Halogen substituiert sein können und wobei weiterhin die heterocyclischen Reste mit einem ankondensierten Benzolkern verbunden sein können.

Als geradkettiges oder verzweigtes C₁-C₄-Alkyl bzw. C₁-C₄-Alkoxy kommen Methyl, Ethyl, Propyl, Isopropyl, Butyl, Isobutyl bzw. Methoxy, Ethoxy, Propoxy, Isopropoxy, Butoxy, Isobutoxy, bevorzugt Methyl bzw. Methoxy in Frage. Halogen ist beispielsweise Fluor, Chlor oder Brom, bevorzugt Chlor.

Bevorzugte aromatische Hydroxyverbindungen für das erfindungsgemäße Verfahren sind solche der Formel

Ar²-OH (II),

worin
- Ar²: Phenyl oder Pyridyl bedeutet, die durch 1 oder 2 Substituenten aus der Gruppe von geradkettigem oder verzeigtem C₁-C₄-Alkyl, geradkettigem oder verzweigtem C₁-C₄-Alkoxy, Phenyl, Cyano und Halogen substituiert sein können und wobei weiterhin Pyridyl mit einem ankondensierten Benzolkern verbunden sein kann.

Besonders bevorzugte aromatische Hydroxyverbindungen für das erfindungsgemäße Verfahren sind solche der Formel

Ar³-OH (III),

worin
- Ar³: Phenyl bedeutet, das durch 1 oder 2 Substituenten aus der Gruppe von geradkettigem oder verzweigtem C₁-C₄-Alkyl, geradkettigem oder verzeigtem C₁-C₄-Alkoxy, Phenyl, Cyano und Halogen substituiert sein kann,
wobei Phenyl bevorzugt 1- oder 2-fach durch Methyl, Ethyl, Propyl, Isopropyl, Methoxy, Ethoxy, Cyano, Fluor, Chlor und/oder Brom substituiert sein kann.

Beispiele für aromatische Monohydroxyverbindungen sind: Phenol, o-, m- und p-Kresol, o-, m- und p-Isopropylphenol, die entsprechenden Halogen- bzw. Alkoxyphenole, wie p-Chlorphenol bzw. p-Methoxyphenol, ferner Monohydroxyverbindungen des Naphtalins, Anthracens und Phenanthrens, weiterhin 4-Hydroxy-pyridin und Hydroxychinolin.

Das erfindungsgemäße Verfahren kann sowohl mit Phosgen als auch mit Chlorkohlensäureestern aromatischer Monohydroxyverbindungen durchgeführt werden. Für den Fall der Durchführung mit Phosgen entsteht zunächst der Chlorkohlensäureester der aromatischen Monohydroxyverbindung, der mit weiterer im Reaktionsgemisch vorhandener aromatischer Monohydroxyverbindung zum symmetrischen Diarylcarbonat dieser aromatischen Monohydroxyverbindung umgesetzt wird. Geht man von Chlorkohlensäureestern und einer aromatischen Monohydroxyverbindung aus, können je nach Estergruppe im Chlorkohlensäureester symmetrische oder unsymmetrische Carbonate erhalten werden. Symmetrische Carbonate erhält man, wenn die im Chlorkohlensäureester enthaltene Estergruppierung und die aromatische Monohydroxyverbindung gleich sind. Da solche symmetrischen Diarylcarbonate jedoch auch direkt aus der aromatischen Monohydroxyverbindung mit Phosgen in der beschriebenen Weise hergestellt werden können, hat ein solches Verfahren nur geringe Bedeutung. Von größerer Bedeutung ist jedoch die Herstellung unsymmetrischer Carbonate aus einer aromatischen Monohydroxyverbindung und einem Chlorkohlensäurester. Hierbei kann die Estergruppe im Chlorkohlensäureester ebenfalls eine aromatische Monohydroxyverbindung sein, die unter den Offenbarungsrahmen für Ar¹-OH(I) bzw. Ar²-OH(II) bzw. Ar³-OH(III) fällt, jedoch im Sinne der Bildung unsymmetrischer Diarylcarbonate ein anderes Substitutionsmuster als die eingesetzte Monohydroxyverbindung aufweist.

Geeignete Chlorkohlensäurester für das erfindungsgemäße Verfahren sind demnach solche der Formel

R¹-OCOCl (IV),

worin R¹ für Ar¹ steht.

Besonders geeignete Chlorkohlensäureester für das erfindungsgemäße Verfahren sind solche der Formel

R²-OCOCl (V),

worin R² für Ar² steht.

In besonders bevorzugter Weise eignen sich für das erfindungsgemäße Verfahren Chlorkohlensäureester der Formel

R³-OCOCl (VI),

worin R³ für Ar³ steht.

Für den Fall, daß Ar¹ bzw. Ar² bzw. Ar³ zweifach substituiert ist, können die beiden Substituenten auch verschiedene aus dem genannten Bedeutungsumfang sein.

Es ist ein wesentliches Merkmal des erfindungsgemäßen Verfahrens, daß es in Gegenwart von Aktivkohle als Katalysator durchgeführt wird. Unter Aktivkohle im Sinne der vorliegenden Erfindung ist aktivierter Kohlenstoff zu verstehen, der aus unterschiedlichen, Kohlenstoff liefernden Vorprodukten hergestellt werden kann. Die Verfahren zur Überführung in die aktive Form können ebenfalls sehr unterschiedlich sein, Bei solchen Herstellungsverfahren werden Aktivkohlen erhalten, die BET-Oberflächen von 200-3.000 m²/g, bevorzugt 300-2.000 m²/g, besonders bevorzugt 500-1.500 m²/g, aufweisen. Als Ausgangsmaterialien zur Herstellung aktivierter Kohlen seien beispielsweise genannt: Sägemehl und andere Holzabfälle, Stroh, verschiedene Kohlensorten, wie Bitumen- oder Braunkohle, Nußschalen, Mineralölteere, Lignin, Polysaccharide, Polyacrylnitril, Knochen, Torf. Ferner können auch Koksprodukte aus Braun- und Steinkohlen eingesetzt werden. In bevorzugter Weise seien genannt: Holz, Cellulose, Lignin, Bitumen- oder Braunkohle, Torf oder Steinkohlenkoks.

Die genannten Kohlenstoff liefernden Vorprodukte können durch verschiedene Methoden aktiviert werden, beispielsweise durch chemische Aktivierung mit Phosphorsäure oder Zinkchlorid, durch Gasaktivierung mit Dampf, Sauerstoff oder Nitrose enthaltenden Gase. Solche voraktivierten Vorprodukte werden sodann thermisch, d.h. durch Verkoken in Aktivkohlen für das erfindungsgemäße Verfahren übergeführt, Diese Herstellungsweisen sind dem Fachmann bekannt und sind genau wie die nähere Beschreibung der dabei erhältlichen verschiedenen Sorten von Aktivkohle in der Literatur ausführlich beschrieben (s. Ullmann's Enzyklopedia of Industrial Chemistry, 5th, Ed., Vol. A5 (1986), S. 124-140 und die dort zitierte Literatur).

Die genannten Aktivkohle-Katalysatoren werden beim Arbeiten mit suspendiertem Katalysator in Rührgefäßen oder Blasensäulen in Mengen von 0,5 bis 100 Gew.-%, bevorzugt von 5 bis 100 Gew.-% und besonders bevorzugt von 5 bis 50 Gew.-%, bezogen auf die eingesetzte Menge an Monohydroxyverbindung, verwendet.

Im Falle einer kontinuierlichen Fahrweise im Gegen- oder Gleichstrom oder in der Rieselphase am Festbettkatalysator werden Katalysatorbelastungen von 0,1 bis 20 g aromatische Hydroxyverbindung pro g Katalysator pro Stunde, bevorzugt von 0,2 bis 10 g · g⁻¹ · h⁻¹ und besonders bevorzugt von 0,2 bis 5 g · g⁻¹ · h⁻¹ verwendet.

Die in diskontinuierlichen Versuchen verwendete Aktivkohle kann bei gleichen Einsatzstoffen ohne Reinigung wiederholt eingesetzt werden. Bei einem Wechsel der Einsatzstoffe wird die Aktivkohle zweckmäßig durch Extrahieren mit inerten Lösungsmitteln, wie sie beispielsweise weiter unten als Reaktionsmedien genannt sind, oder mit Alkoholen, wie Methanol, Ethanol, Isopropanol oder Butanol, mit Estern oder Amiden der Essigsäure oder durch Behandlung mit überhitztem Wasserdampf gereinigt.

Bei kontinuierlicher Arbeitsweise kann die eingesetzte Aktivkohle über lange Zeit im Reaktor verbleiben. Eine Regenerierung lohnt sich in der Regel nicht; sie kann jedoch durch Überleiten von überhitztem Wasserdampf gegebenenfalls unter Zugabe von untergeordneten Mengen an Luft (etwa 0,1 bis 20 Gew.-%, bezogen auf die eingesetzte Wasserdampfmenge) bei 150 bis 800°C oder durch Überleiten von 0,01 bis 5 Gew.-% Sauerstoff enthaltenden Verdünnungsgasen,wie Stickstoff, Kohlenmonoxid oder Kohlendioxid oder durch Kohlendioxid allein bei 200 bis 800°C erfolgen. Die bevorzugte Regenerierungstemperatur liegt bei 250-700°C, besonders bevorzugt bei 250 bis 600°C.

Das erfindungsgemäße Verfahren wird bei einer Temperatur im Bereich von 50-350°C, bevorzugt 100-300°C, besonders bevorzugt 100-250°C durchgeführt. Während der Durchführung des erfindungsgemäßen Verfahrens kann die Temperatur im genannten Bereich verändert, in bevorzugter Weise erhöht werden.

Das erfindungsgemäße Verfahren wird bei einem Druck im Bereich von 0,5-20 bar, bevorzugt 0,8-10 bar, besonders bevorzugt 1,0-5 bar durchgeführt. Die Einhaltung eines bestimmten Druckes ist für den Erfolg des erfindungsgemäßen Verfahrens von untergeordneter Bedeutung.

Man kann das erfindungsgemäße Verfahren unter Mitwirkung von Lösungsmitteln durchführen. Dies empfiehlt sich, wenn die Ausgangs- oder Endprodukte hohe Schmelzpunkte aufweisen. Geeignet sind Lösungsmittel, die unter den Reaktionsbedingungen inert sind. Hierzu seien beispielsweise genannt: aliphatische und aromatische Kohlenwasserstoffe, wie Pentan, Hexan, Octan, Decan Isononan, Isooctan, Cyclohexan, tert.-Butyl-cyclohexan, Cyclododecan, Benzol, die isomeren Xylole, Diethylbenzol, Diisopropylbenzol, Cumol, Alkylnaphthaline, Biphenyl; halogenierte Kohlenwasserstoffe, wie Dichlormethan, Trichlorethylen, Tetrachlorethylen, Dichlorethan, Dichlorhexan, Chlorbenzol, Dichlorbenzol, Trichlorbenzol, Chlornaphthalin, Dichlornaphthalin; stabile Ether, wie Diphenylether und Ditolylether. Weitere einsetzbare inerte Lösungsmittel sind dem Fachmann geläufig.

Vorzugsweise wird das Verfahren jedoch in der Schmelze durchgeführt, beispielsweise, indem man in eine Suspension von Aktivkohle in einer Schmelze der aromatischen Monohydroxyverbindung Phosgen oder einen Chlorkohlensäureester der genannten Art einleitet und nach Beendigung der Reaktion den Katalysator durch Filtration abtrennt. Das aus dem Reaktionsgefäß mit dem gebildeten Chlorwasserstoff entweichende Phosgen oder entweichender Chlorkohlensäureester kann in eine weitere Suspension von Aktivkohle in der aromatischen Monohydroxyverbindung eingeleitet werden, wobei auch mehrere solcher weiterer Suspensionen angefügt werden können, bis das Phosgen, bzw. der Chlorkohlensäureester völlig verbraucht ist und nur noch Chlorwasserstoff austritt. Auf diese Weise kann eine Carbonatsynthese nach dem erfindungsgemäßen Verfahren technisch beispielsweise in einer Rührkesselkaskade durchgeführt werden, in der der Gasstrom dem Flüssigkeitsstrom entgegengeführt wird.

Eine weitere bevorzugte Ausführungsform der Synthese ist die Begasung einer Schmelze der aromatischen Monohydroxyverbindung mit suspendiertem Aktivkohlekontakt mit Phosgen oder Phosgen-Chlorwasserstoff-Gemischen oder mit flüchtigen Chlorkohlensäureestern in einer kontinuierlich arbeitenden Blasensäule, die mit weiteren Blasensäulen ebenfalls zu einer Kaskade vereinigt werden kann, in der Phosgen bzw. Chlorkohlensäureester und Schmelze im Gegenstrom miteinander umgesetzt werden, etwa in der Weise, daß frisches Phosgen oder frischer Chlorkohlensäureester in die letzte Säule einer Kaskade eingespeist wird, aus der dann das fertige rohe Carbonat entnommen wird, während der partiell umgesetzte, Chlorwasserstoff enthaltende Phosgen- oder Chlorkohlensäureester-Strom in die noch überschüssige aromatische Hydroxyverbindung enthaltenden Säulen eingeblasen wird, dort weiter an Phosgen bzw. Chlorkohlensäureester verarmt, bis aus der ersten Säule der Kaskade, in die frische aromatische Hydroxyverbindung eindosiert wird, nur noch Chlorwasserstoff entweicht.

Eine weitere bevorzugte Durchführungsform ist das Gleichstromverfahren, bei dem aromatische Hydroxyverbindung und Phosgen bzw. Chlorkohlensäureester im Gleichstrom beispielsweise von oben her auf eine in einem Rohr angeordnete Katalysatorschüttung aufgebracht und unten am Fuß des Rohres Chlorwasserstoff und Phosgenierprodukte an gleicher Stelle oder räumlich getrennt abgezogen werden.

Eine noch weitere bevorzugte Ausführungsform mit besonders günstigen Ergebnissen ist die Durchführung der erfindungsgemäßen Umsetzung in der Rieselphase, wobei die aromatische Monohydroxyverbindung als Schmelze oder in Form einer Lösung oben auf ein Bett von stückiger Aktivkohle gegeben wird und diesem Flüssigkeitsstrom von unten ein Strom von Phosgen oder flüchtigem Chlorkohlensäureester entgegengeschickt wird. Zweckmäßig wird diese Ausführungsform in einer Kolonne durchgeführt, die auch Zwischenböden zur verbesserten Verteilung von Gas- und Flüssigkeitsstrom enthalten kann. Auch bei dieser Durchführung in der Rieselphase kann der entweichende Gasstrom, der an Phosgen oder flüchtigem Chlorkohlensäureester abgereichert ist und bereits Chlorwasserstoff enthält, einer folgenden Rieselphasenkolonne zur weiteren Umsetzung in der bereits beschriebenen Art zugeführt werden.

Im Rahmen der genannten Ausführungsformen und unter Benutzung der ebenfalls genannten Reaktionsapparate stellt man beide Stoffströme zweckmäßigerweise so ein, daß beispielsweise am Boden einer Kolonne die aromatische Monohydroxyverbindung vollständig umgesetzt ist und am oberen Ende kein Phosgen oder Chlorkohlensäureester mehr austritt. Dies kann sich in der besprochenen Weise auf einen einzelnen Reaktionsapparat, aber auch auf eine kaskadenartige Anordnung von mehreren Reaktionsapparaten beziehen.

Bei der Gleichstromfahrweise insbesondere von schwer flüchtigen Chlorkohlensäureestern kann gegebenenfalls von unten ein schwacher Inertgasstrom (beispielsweise Kohlendioxid, Stickstoff, Erdgas u.a.) entgegengeschickt werden, um entstehenden Chlorwasserstoff auszublasen.

Das Verhältnis der Reaktionspartner weicht im allgemeinen wenig von den äquivalenten Mengen ab, d.h. die aromatische Monohydroxyverbindung wird in der Regel mit Phosgen im molaren Verhaltnis von 1,5-3:1, bevorzugt 1,8-2,5:1 umgesetzt; das äquivalente Verhältnis ist in diesem Fall 2:1. In entsprechender Weise wird die aromatische Monohydroxyverbindung mit einem Chlorkohlensäureester im Molverhältnis von 0,5-1,5:1, bevorzugt 0,8-1,5:1 umgesetzt, wobei in diesem Fall das molare Verhältnis 1:1 den äquivalenten Mengen entspricht.

Diese Verhältnisse lassen sich durch die folgenden Reaktionsgleichungen ausdrücken:

2 Ar¹-OH + COCl₂ → Ar¹-OCOO-Ar¹ + 2HCl (VII)

bzw.

Ar¹-OH + ClCOO-R¹ → Ar¹-OCOO-R¹ + HCl (VIII)

Die weitgehende Umsetzung beider Reaktionspartner (aromatische Monohydroxyverbindung und Phosgen bzw. Chlorkohlensäureester) durch möglichst geringe Abweichung von den Äquivalenzmengen kann in einer dem Fachmann bekannten Weise durch Abstimmung der Molverhältnisse, der Verweilzeiten am Kontakt und der Reaktionstemperatur gesteuert werden.

Das auf diese Weise gewonnene rohe aromatische Carbonat ist häufig schon sehr rein und kann nach Entgasung von restlichem Chlorwasserstoff oder anderen flüchtigen Stoffen bereits in dieser Form für viele Zwecke verwendet werden, da insbesondere eine Katalysatorabtrennung nicht mehr erforderlich ist. Für anspruchsvollere Verwendungen kann das Carbonat wie üblich durch Destillation oder Kristallisation weiter gereinigt werden.

### Beispiele

### Beispiel 1

In einem Reaktionsrohr von ca. 4 cm Durchmesser wurden 200 g (2,13 Mol) Phenol in Gegenwart von 20 g pulverisierter Aktivkohle über eine Fritte am Boden des Rohres mit einem schwachen Phosgenstrom begast. Nach etwa 2 h bei 150°C wurde ein Phenolumsatz von 23 % gefunden, eine Selektivität zu Phenylchlorkohlensäureester von 84 % und zu Diphenylcarbonat von 16 %.

### Beispiel 2

Beispiel 1 wurde wiederholt; es wurde 2 h bei 180°C mit Phosgen begast. Der Umsatz an Phenol betrug 41 %, die Selektivität zu Phenylchlorkohlensäureester 24 % und zu Diphenylcarbonat 76 %.

### Beispiel 3

Das Reaktionsgemisch aus Beispiel 2 wurde weitere 60 min bei 180°C ohne Phosgenbegasung gerührt. Dabei ging der Phenylchlorkohlensäureester mit noch vorhandenem Phenol in Diphenylcarbonat über; die Selektivitat zu Diphenylcarbonat war praktisch quantitativ.

### Beispiel 4

Ein beheizbares Reaktionsrohr von ca. 30 cm Länge und 2,8 cm Durchmesser wurde mit ca. 90 g Aktivkohle einer Kornung von 4 mm (Typ VFTC 40/4 (Formkohle) des Bergwerkverbandes GmbH, Verkaufsgesellschaft für Teererzeugnisse, Duisburg) gefüllt und von oben mit einem Strom von 50 g/h (0,53 Mol) Phenol und im Gegenstrom mit 30 g/h (0,30 Mol) Phosgen bei 160°C beschickt. Am Fuß dieser Kolonne ließ sich nach einer Einstellzeit von 1 h laufend ein Gemisch entnehmen, das einen Phenolumsatz vn 42 %, eine Selektivität zu Diphenylcarbonat von 57 % und eine Selektivität zu Phenylchlorameisensäureester von 43 % aufwies.

Ließ man dieses Gemisch bei 170°C mit einer Geschwindigkeit von 50 g/h über eine zweite Katalysatorschüttung der oben beschriebenen Art fließen, so war die Umwandlung in Diphenylcarbonat quantitativ.

### Beispiel 5

Ein beheizbares Reaktionsrohr von etwa 150 cm Länge und 2,8 cm Durchmesser wurde mit der Aktivkohle aus Beispiel 4 gefüllt und von oben mit einem Phenolstrom von 40 g/h und im Gegenstrom mit 30 g/h Phosgen beschickt, das ca. 30 cm über dem unteren Ende der Kohleschüttung eingedüst wurde. Nach einer Einstellzeit von etwa 6 h floß am unteren Ende des Rohres ein Produkt ab, das zu über 98 % aus Diphenylcarbonat bestand. Die Selektivität zu Diphenylcarbonat war praktisch quantitativ.

### Beispiel 6 (zum Vergleich):

Das Beispiel 1 wurde ohne Zusatz von Aktivkohle wiederholt. Nach 4 h bei 175°C betrug der Phenolumsatz < 0,2 %.

## Patentansprüche

1. Verfahren zur Herstellung von Arylcarbonaten durch Umsetzung von aromatischen Monohydroxyverbindungen mit Phosgen oder Chlorkohlensäureestern aromatischer Monohydroxyverbindungen, dadurch gekennzeichnet, daß man die Umsetzung bei einer Temperatur im Bereich von 50-350°C in Gegenwart von Aktivkohle als Katalysator durchführt.

2. Verfahren nach Anspruch 1, dadurch gekennzeichnet, daß man als aromatische Monohydroxyverbindung eine der Formel
Ar¹-OH
einsetzt, worin
Ar¹ Phenyl, Naphtyl, Anthryl, Phenanthryl, Indanyl, Tetrahydronaphthyl oder den Rest eines 5- oder 6-gliedrigen aromatischen Heterocyclus mit 1 oder 2 Heteroatomen aus der Gruppe von N, O und S bedeutet, wobei diese isocyclischen oder heterocyclischen Reste durch 1 oder 2 Substituenten aus der Gruppe von geradkettigem oder verzweigtem C₁-C₄-Alkyl, geradkettigem oder verzweigtem C₁-C₄-Alkoxy, Phenyl, Cyano und Halogen substituiert sein können und wobei weiterhin die heterocyclischen Reste mit einem ankondensierten Benzolkern verbunden sein können.

3. Verfahren nach Anspruch 2, dadurch gekennzeichnet, daß man als aromatische Hydroxyverbindung eine solche der Formel
Ar²-OH
einsetzt, worin
Ar² Phenyl oder Pyridyl bedeutet, die durch 1 oder 2 Substituenten aus der Gruppe von geradkettigem oder verzeigtem C₁-C₄-Alkyl, geradkettigem oder verzweigtem C₁-C₄-Alkoxy, Phenyl, Cyano und Halogen substituiert sein können und wobei weiterhin Pyridyl mit einem ankondensierten Benzolkern verbunden sein kann.

4. Verfahren nach Anspruch 1, dadurch gekennzeichnet, daß als Chlorkohlensäureester einer der Formel
R¹-OCOCl
eingesetzt wird, worin
R¹ für Ar¹ steht.

5. Verfahren nach Anspruch 4, dadurch gekennzeichnet, daß man als Chlorkohlensäureester einen der Formel
R²-OCOCl
eingesetzt, worin
R² für Ar² steht.

6. Verfahren nach Anspruch 1, dadurch gekennzeichnet, daß man bei einer Temperatur im Bereich von 100-300°C, bevorzugt 100-250°C arbeitet.

7. Verfahren nach Anspruch 1, dadurch gekennzeichnet, daß man bei einem Druck im Bereich von 0,5-20 bar, bevorzugt 0,8-10 bar, besonders bevorzugt 1-5 bar arbeitet.

8. Verfahren nach Anspruch 1, dadurch gekennzeichnet, daß das molare Verhältnis der aromatischen Monohydroxyverbindung zu Phosgen 1,5-3:1, bevorzugt 1,8-2,5:1, oder das molare Verhältnis der aromatischen Monohydroxyverbindung zu Chlorkohlensäureester 0,5-1,5:1, bevorzugt 0,8-1,5:1 beträgt.

9. Verfahren nach Anspruch 1, dadurch gekennzeichnet, daß eine Aktivkohle mit einer BET-Oberfläche von 200-3.000 m²/g, bevorzugt 300-2.000 m²/g, besonders bevorzugt 500-1.500 m²/g in einer Menge von 0,5 - 100 Gew.-%, bezogen auf die Menge der Monohydroxyverbindung, bei suspendierten Katalysator bzw. mit Belastungen von 0,1 - 20 g pro g Katalysator pro Stunde bei der Anordnung als Festbettkatalysator eingesetzt wird.

10. Verfahren nach Anspruch 1, dadurch gekennzeichnet, daß eine Aktivkohle eingesetzt wird, die aus Sägemehl und anderen Holzabfällen, Stroh, Kohlensorten, Nußschalen, Mineralölteeren, Lignin, Polysacchariden, Polyacrylnitril, Knochen, Torf oder Koksprodukte aus Braun- oder Steinkohlen, bevorzugt aus Holz, Cellulose, Lignin, Bitumen- oder Braunkohle, Torf oder Steinkohlenkoks hergestellt wurde.

## Claims

1. A process for the production of aryl carbonates by reaction of aromatic monohydroxy compounds with phosgene or chlorocarbonic acid esters of aromatic monohydroxy compounds, characterized in that the reaction is carried out at a temperature of 50 to 350°C in the presence of active carbon as catalyst.

2. A process as claimed in claim 1, characterized in that the aromatic monohydroxy compound used corresponds to the following formula
Ar¹-OH (I)
in which
Ar¹ represents phenyl, naphthyl, anthryl, phenanthryl, indanyl, tetrahydronaphthyl or the residue of a 5- or 6-membered aromatic heterocycle containing 1 or 2 hetero atoms from the group consisting of N, O and S; these isocyclic or heterocyclic residues may be substituted by 1 or 2 substituents from the group consisting of linear or branched C₁₋₄ alkyl, linear or branched C₁₋₄ alkoxy, phenyl, cyano and halogen and the heterocyclic residues may be attached to a fused benzene ring.

3. A process as claimed in claim 2, characterized in that the aromatic hydroxy compound used corresponds to the following formula
Ar²-OH (II)
in which
Ar² represents phenyl or pyridyl which may be substituted by 1 or 2 substituents from the group consisting of linear or branched C₁₋₄ alkyl, linear or branched C₁₋₄ alkoxy, phenyl, cyano and halogen; in addition, the pyridyl may be attached to a fused benzene ring.

4. A process as claimed in claim 1, characterized in that the chlorocarbonic acid ester used corresponds to the following formula
R¹-OCOCl
in which R¹ represents Ar¹.

5. A process as claimed in claim 4, characterized in that the chlorocarbonic acid ester used corresponds to the following formula
R²OCOCl
in which R² represents Ar².

6. A process as claimed in claim 1, characterized in that it is carried out at a temperature of 100 to 300°C and preferably at a temperature of 100 to 250°C.

7. A process as claimed in claim 1, characterized in that it is carried out under a pressure of 0.5 to 20 bar, preferably under a pressure of 0.8 to 10 bar and more preferably under a pressure of 1 to 5 bar.

8. A process as claimed in claim 1, characterized in that the molar ratio of the aromatic monohydroxy compound to phosgene is 1.5-3:1 and preferably 1.8-2.5:1 or the molar ratio of the aromatic monohydroxy compound to chlorocarbonic acid ester is 0.5-1.5:1 and preferably 0.8-1.5:1.

9. A process as claimed in claim 1, characterized in that an active carbon having a BET surface of 200 to 3,000 m²/g, preferably 300 to 2,000 m²/g and more preferably 500 to 1,500 m²/g is used in a quantity of 0.5 to 100% by weight, based on the quantity of monohydroxy compound, where the catalyst is suspended or with loads of 0.1 to 20 g per g catalyst per hour where the catalyst is arranged in the form of a fixed bed.

10. A process as claimed in claim 1, characterized in that the active carbon used is made from sawdust and other wood waste, straw, various types of coal, nutshells, mineral oil tars, lignin, polysaccharides, polyacrylonitrile, bones, peat or coke products of lignite and mineral coals and preferably of wood, cellulose, lignin, bitumen coal or lignite, peat or mineral coal coke.

## Revendications

1. Procédé pour la préparation de carbonates d'aryle par réaction de composés monohydroxylés aromatiques avec le phosgène ou des esters chlorocarboniques de composés monohydroxylés aromatiques, caractérisé en ce que l'on effectue la réaction à une température dans l'intervalle de 50 à 350°C en présence de charbon actif qui sert de catalyseur.

2. Procédé selon revendication 1, caractérisé en ce que l'on utilise en tant que composé monohydroxylé aromatique un composé de formule
Ar¹-OH
dans laquelle
Ar¹ représente un groupe phényle, naphtyle, anthryle, phénanthryle, indanyle, tétrahydronaphtyle ou le radical d'un hétérocycle aromatique à 5 ou 6 chaînons contenant un ou deux hétéroatomes choisis parmi N, O et S, ces radicaux isocycliques ou hétérocycliques pouvant porter un ou deux substituants choisis parmi les groupes alkyle à chaîne droite ou ramifiée en C₁-C₄, les groupes alcoxy à chaîne droite ou ramifiée en C₁-C₄, les groupes phényle, cyano et les halogènes, les radicaux hétérocycliques pouvant en outre être condensés avec un cycle benzénique.

3. Procédé selon revendication 2, caractérisé en ce que l'on utilise en tant que composé hydroxylé aromatique un composé de formule
Ar²-OH
dans laquelle
Ar² représente un groupe phényle ou pyridyle qui peut porter un ou deux substituants choisis parmi les groupes alkyle à chaîne droite ou ramifiée en C₁-C₄, les groupes alcoxy à chaîne droite ou ramifiée en C₁-C₄, les groupes phényle, cyano et les halogènes, le groupe pyridyle pouvant en outre être condensé avec un cycle benzénique.

4. Procédé selon revendication 1, caractérisé en ce que l'on utilise en tant qu'ester chlorocarbonique un composé de formule
R¹-OCOCl
dans laquelle
R¹ a les mêmes significations que Ar¹.

5. Procédé selon revendication 4, caractérisé en ce que l'on utilise en tant qu'ester chlorocarbonique un ester de formule
R²-OCOCl
dans laquelle
R² a les mêmes significations que Ar².

6. Procédé selon revendication 1, caractérisé en ce que l'on opère à une température dans l'intervalle de 100 à 300°C, de préférence de 100 à 250°C.

7. Procédé selon revendication 1, caractérisé en ce que l'on opère à une pression dans l'intervalle de 0,5 à 20 bar, de préférence de 0,8 à 10 bar et plus spécialement de 1 à 5 bar.

8. Procédé selon revendication 1, caractérisé en ce que le rapport molaire entre le composé monohydroxylé aromatique et le phosgène va de 1,5 à 3 : 1, de préférence de 1,8 à 2,5 : 1, ou bien le rapport molaire entre le composé monohydroxylé aromatique et l'ester chlorocarbonique va de 0,5 à 1,5 : 1, de préférence de 0,8 à 1,5 : 1.

9. Procédé selon revendication 1, caractérisé en ce que l'on utilise un charbon actif présentant une surface BET de 200 à 3 000 m²/g, de préférence de 300 à 2 000 m²/g et plus spécialement de 500 à 1 500 m²/g en quantité de 0,5 à 100 % du poids du composé monohydroxylé dans le cas d'un catalyseur en suspension ou avec des charges de 0,1 à 20 g/g de catalyseur et par heure dans le cas d'un catalyseur disposé en lit fixe.

10. Procédé selon revendication 1, caractérisé en ce que l'on utilise un charbon actif qui a été obtenu à partir de farine de bois et d'autres déchets de bois, de paille, de divers types de charbon, de coques de noix, de goudron de pétrole, de lignine, de polysaccharides, de polyacrylonitrile, d'os, de tourbe ou de coke de lignite ou de houille, de préférence de bois, de cellulose, de lignine, de charbon de bitume ou de lignite, de tourbe ou de coke de houille.
